(19) **Europäisches Patentamt European Patent Office Office européen des brevets**

(11) **EP 4 670 748 A1**

(12) # EUROPEAN PATENT APPLICATION

(43) Date of publication:
**31.12.2025 Bulletin 2026/01**

(21) Application number: **24184995.9**

(22) Date of filing: **27.06.2024**

(51) International Patent Classification (IPC):
*A61L 27/48* (2006.01)    *A61L 27/54* (2006.01)

(52) Cooperative Patent Classification (CPC):
(C-Sets available)
**A61L 27/48; A61L 27/54;** A61L 2430/16    (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Universität zu Köln**
  **50931 Köln (DE)**
• **Julius-Maximilians-Universität Würzburg**
  **97070 Würzburg (DE)**

(72) Inventors:
• **ZWINGELBERG, Sarah**
  **50996 Köln (DE)**
• **LANG, Gregor**
  **97070 Würzburg (DE)**

(74) Representative: **Viering, Jentschura & Partner mbB**
  **Patent- und Rechtsanwälte**
  **Hamborner Straße 53**
  **40472 Düsseldorf (DE)**

(54) **A MEDICAL IMPLANT WITH FIBER RE-INFORCED CHITOSAN FILM MATRIX**

(57)    The present invention provides a medical implant, comprising (a) a matrix layer comprising chitosan; and (b) a reinforcement structure comprising polymer filaments or polymer fibers, whereby the reinforcement structure is incorporated into the matrix layer. The invention further relates to the medical implant for use in the treatment of ophthalmological diseases or skin disorders. The invention finally relates to a method for producing the medical implant of the invention.

Fig. 6

**(Cont. next page)**

(52) Cooperative Patent Classification (CPC): (Cont.)

C-Sets
**A61L 27/48, C08L 5/08;**
**A61L 27/48, C08L 29/04;**
**A61L 27/48, C08L 67/04;**
**A61L 27/48, C08L 89/06**

**Description**

The present invention provides a medical implant, comprising (a) a matrix layer comprising chitosan; and (b) a reinforcement structure comprising polymer filaments or polymer fibers, whereby the reinforcement structure is incorporated into the matrix layer. The invention further relates to the medical implant for use in the treatment of ophthalmological diseases or skin disorders. The invention finally relates to a method for producing the medical implant of the invention.

BACKGROUND OF THE INVENTION

Corneal diseases are the third leading cause of blindness worldwide. The medical relevance of artificial corneas is gaining importance, not at least due to the SARS-CoV-2 pandemic. A multicenter study from 26 European countries with 110 participating cornea banks has highlighted the rapidly growing donor shortage in 2020: it showed an average monthly donor decline of 38%, 68%, and 41% in month-to-month comparisons from February 2018 to May 2020. In January 2023, 1667 patients were on the waiting list for corneal transplantation in Cologne alone. This highlights the urgent need to replace the apparent donor shortage with artificial corneal transplants and to intensify and promote research in this field. Worldwide the situation is even more dramatic, especially since there are many countries without regulated access to corneal transplantation. The reasons for corneal transplantation are manifold. Possible causes are acute and chronic infections, previous trauma, corneal dystrophies, and degenerative diseases.

Therapy-resistant erosions and ulcerations can be caused by infectious events, e.g., bacteria (often from contact lenses), viruses (e.g., HSV, CMV, VZV), fungal infections (e.g., Candida, Aspergillus), pathogens such as Acanthamoeba and Pseudomonads, or trauma. These infections, particularly with herpes viruses, can result in the development of neurotrophic keratopathy, which is associated with decreased innervation of the cornea.

In therapy-resistant erosions and ulcerations, amniotic grafts along with corneal replacement procedures are clinically used to promote healing. In such defects, membrane thicknesses between 15 and 50 $\mu$m are sufficient because the membrane does not require mechanical stability and does not necessarily need to be optically clear. Amniotic membrane represents the innermost layer of the placenta and has epithelial proliferative, anti-angiogenic and anti-inflammatory effects via various factors, which is used for wound healing in corneal injuries. It has the disadvantage that amniotic membrane has to be obtained by live donation of placenta from women in childbirth.

A further disadvantage is given by the fact that, after the amniotic membrane is sewn onto corneal defects, the effect of the factors diminishes significantly within a few days, making them less efficient in supporting tissue healing.

If anterior stromal scarring develops in the setting of inflammation, anterior keratoplasty (deep anterior lamellar keratoplasty, DALK) may be required, and penetrating keratoplasty (PKP) may be required for deep stromal scarring. Anterior keratoplasty may also be required for non-infectious conditions, such as traumatic scars, or for conditions such as keratoconus, which is common in the population. Keratoconus (KC) is a degenerative disease of the cornea. KC leads to a progressive central thinning of the cornea, with a decrease in central pachymetry at the apex and an increase in $K_{max}$ in pentacam (combined device consisting of a slit illumination system and a Scheimpflug camera, which rotate together around the eye) with concomitant progressive myopia and astigmatism. In advanced cases KC is no longer correctable with conventional conservative methods (contact lens fitting) in severe cases, resulting in relevant visual impairment in, mostly young, patients.

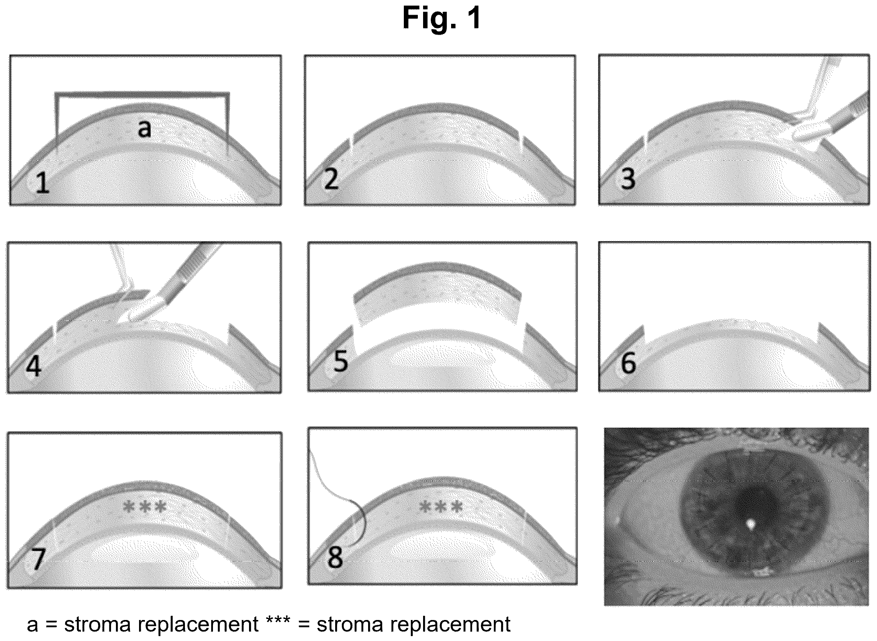

Keratoconus can be stopped by crosslinking if the central corneal thickness is sufficient. However, in cases of reduced pachymetry values from about 400 $\mu$m and progressive corneal thinning, DALK is useful, as well as when stromal scarring forms at the apex as a result of corneal thinning, leading to a decrease in visual acuity. In cases where an anterior lamellar corneal graft or even a complete corneal graft is required, the stromal substitute must not only be optically clear but also have adequate mechanical stability. For a lamellar layer transplant, the membrane must have a thickness of 300-350 $\mu$m and for a complete corneal graft about 550 $\mu$m (see Figure 1). In corneal transplant procedures, it is important to consider that limbal vascularization, which can occur in the context of corneal hypoxia (in the context of infection and previous trauma) leads to an increased expression of HIF-1alpha and consequently increased VEGF release. Also, limbal stem cell insufficiency leads to a corneal vascularization. Both poses an increased risk for graft rejection and vision loss. In these cases, anti-VEGF (bevacizumab) can be applied and fine needle diathermy can be performed as part of this high-risk keratoplasty, but the effect is short-lasting. Currently, HLA typed keratoplasty is preferred in these cases, although the waiting time for such a graft is significantly longer than for a regular corneal transplant. The waiting time can be several years, during which time a progressive decrease of visual acuity can be expected.

It is to note, that in recent past, there are various publications demonstrating, that not the material properties alone, but also their topological features significantly tailor cell behaviour in terms of adhesion, differentiation, proliferation, and migration calling for new implants providing hierarchical and topological structures that promote cell-biological performance. This hypothesis is further underscored by the fact, that the cornea is a highly hierarchical, five-layer tissue with predominantly anisotropic cell/ECM arrangement.

Hence, there is still a need for the efficient and safe treatment of ophthalmological diseases and especially corneal diseases.

The objective of the present invention thus is to provide an improvement or an alternative to the prior art.

This problem is solved by provision of a medical implant according to claims 1 to 10, the use of the medical implant according to claims 11 to 12, and a method for producing a medical implant according to claims 13 to 15. Specific embodiments are subject matter of further dependent claims.

SUMMARY OF THE INVENTION

A first aspect of the invention provides a medical implant, comprising: (a) a matrix layer comprising chitosan; and (b) a reinforcement structure comprising polymer filaments or polymer fibers, whereby the reinforcement structure is incorporated into the matrix layer.

The medical implant of the first aspect combines many advantages of cornea graft therapies while avoiding the disadvantages of said therapies.

At first, chitosan combines a spectrum of attractive properties such as anti-inflammatory, antimicrobial, antioxidant, antitumoral and wound healing features.

The chitosan-based implant is non-toxic, possess good transparency, good biocompatibility, good permeability and suitable mechanical properties with corneal tissue and has the potential to promote cell growth.

A chitosan-based implant can be produced rather easily, e.g. by simple film casting. As the inventors have shown, the chitosan matrix can be modified to provide more biomimetic patterned surfaces in order to reproduce the nature architecture *in vivo.*

Furthermore, chitosan can be combined/modified with various natural materials such as keratin, silk fibroin, collagen or gelatine as well as with synthetic materials such as polycaprolactone, polyethylene glycol or silicone.

Although the chitosan film matrix of the invention has a sufficient pressure resistance, as demonstrated herein by the determination of burst pressure, it showed weak performance in the sewability-test irrespective of the film thickness as the thread would tear out easily. However, it was found that the additional reinforcement of the chitosan matrix by a reinforcement structure comprising polymer filaments or polymer fibers improved the sewability and in the same time retained the positive effects of the chitosan matrix. As a result, the chitosan film layer with the embedded reinforcement structure provides a basic composite structure, which can be further modified (e.g. by coating, surface patterning or addition of a further hydrogel layer) in order to mimic the highly hierarchical, multi-layer tissue of the cornea.

In sum, the medical implant of the invention has the potential to provide a well-balanced combination of materials and morphologies to address biological, optical, and mechanical design criteria.

DETAILED DESCRIPTION OF THE INVENTION

**The matrix layer**

The medical implant relates to a fiber/textile-reinforced matrix layer comprising chitosan. The chitosan matrix represents a dried chitosan hydrogel and thereby result in a chitosan film. Accordingly, the terms "matrix" or "matrix layer" as used herein are synonymous to the terms "film matrix" or "film matrix layer".

Advantageously, in the context of the invention, it may be provided that the chitosan is a low molecular weight (LMW) chitosan with an average Mw of less than 250 KDa and preferably of less than 100 kDa. The use of a LMW chitosan is advantageous, insofar it has a high solubility and can generate stable and transparent films.

In a preferred embodiment, the chitosan is a medical grade Chitosan, which exhibits a purity of > 97%, a heavy metal content of <1 ppm, Arsenic content <0,2 ppm, and a degree of deacetylation $\geq$ 95 %.

It may be further provided that the chitosan preferably a degree of deacetylation of at least 95%, more preferably of at least 97%, and even more preferably of at least 99%, as determined by $^1$H-NMR analysis. This high degree of acetylation has shown to increase the solubility and decrease the viscosity of the respective chitosan solution and thereby facilitates the production of the implant. The low viscosity is of special importance when producing a chitosan film matrix with micro-pattern containing surface structure enabling the adhesion and migration of epithelial cells. Due to the free amino groups formed by deacetylation, it is a polycation with a high charge density in a non-alkaline solution. It is non-toxic, antibacterial, antiviral and antiallergenic.

In a further implementation, the medical implant of the invention is characterized in that the matrix layer comprises a blend of chitosan and collagen which is crosslinked, preferably by use of glutaraldehyde, NaOH, or carbodiimides such as 1-Ethyl-3-(3-dimethylaminopropyl) carbodiimide (EDC).

The crosslinked blend of chitosan and collagen has the advantage that collagen displays cell adhesive sites rendering the structure more attractive for adhesion and migration of cells. Chitosan however, is more transparent and known to inhibit biofilm formation.

In an alternative implementation, the medical implant of the invention is characterized in that the matrix layer comprises crosslinked chitosan, preferably crosslinked by use of glutaraldehyde.

The crosslinked chitosan has the advantage that it provides transparency, mechanical integrity and inhibits biofilm formation, whereas it can be loaded with bioactive drugs.

In a preferred embodiment the surface of the chitosan matrix layer is preferably coated with a fibrinogen or an RGD-containing peptide. Arginylglycylaspartic acid (RGD) is the most common peptide motif responsible for cell adhesion to the extracellular matrix (ECM). Cell adhesion proteins called integrins recognize and bind to this sequence, which is found within many matrix proteins, including fibronectin, fibrinogen, vitronectin, osteopontin, and several other adhesive extracellular matrix proteins. Accordingly, ECM-derived ligands such as RGD can be used to control cellular responses to a biomaterial, and it is submitted that the addition of RGD can improve cell attachment, alignment, proliferation, and ECM protein expression.

In a more preferred embodiment, the surface of the chitosan matrix layer is coated with the tetrapeptide RGDS. The coating is typically performed by a covalent bonding. Several methods are known by the skilled person to achieve this covalent linkage, whereby this linkage of the layer surface to the peptide RGDS is preferably performed via carbodiimide reaction.

According to one embodiment, the film matrix layer has a chitosan concentration of between 50 and 99 wt.%. Layers with chitosan concentration of this range have been shown to possess beneficial mechanical properties. It is to note that the above defined chitosan concentration relates to the concentration of the dried chitosan hydrogel generating a chitosan film (also denominated herein as chitosan matrix or chitosan film matrix) and not to the concentration of the chitosan as constituent of the hydrogel used for the mold casting.

According to a further embodiment, the matrix layer (i.e., the final film matrix) has a thickness of between 10 and 800 $\mu$m. The thickness can be easily adjusted by the preferred film casting method and can be specifically adapted to the therapeutic use.

According to one embodiment, the matrix layer has a diameter of between 3 and 30 mm.

According to a further embodiment, the matrix layer has a thickness of between 10 and 800 $\mu$m. For an anterior lamellar corneal graft, the matrix layer must have a thickness of 300 to 350 $\mu$m and for a complete corneal graft about 550 $\mu$m.

According to one embodiment, the matrix layer has a transparency of at least 75% and preferably of at least 80%. The preferred use a corneal graft requires such a high transparency, at least in the central area to ensure good eyesight.

According to a further embodiment, the matrix layer further comprises an additive such as polyethylene oxide (PEO), silk proteins, cellulose nanofibrils, or tannic acid. By using these additives, the rheological properties of the chitosan matrix layer can be modified for improved preparation, handling or *in vivo* behavior.

According to a preferred embodiment, the matrix layer further comprises a drug or a bioactive molecule, preferably selected from the group consisting of curcumin, collagen I, EGF, betacellulin, anti-VEGF, rho-associated coiled-coil-containing protein kinase (ROCK) inhibitor, cyclo-trans-4-L-hydroxypropyl-L-serine (JPB485), human recombinant nerve growth factor (NGF), Insulin growth factors, Bosentan, Sparsentan, 4-Hydroxytamoxifen, Tamoxifen and estrogen receptor $\beta$ (ER-$\beta$) agonist LY500307, and combinations thereof.

Rho-Kinase (ROCK) Inhibitors are already successfully applied in eye drops. Rho-kinase (ROCK) signaling pathways control basic cellular functions, making ROCK an important therapeutic target in various corneal pathophysiologic conditions. Rock inhibitors can therefore be used as pharmacological additive in artificial cornea membranes.

Curcumin has shown antimicrobial properties in many studies as well as neuroprotective effects, which is a very important factor in corneal diseases such as neurotrophic keratopathy. Neurotrophic keratopathy is most commonly caused by herpetic keratitis but can also be caused by any other disease that disrupts the normal innervation of the cornea.

A placenta extract-derived dipeptide (JBP485) significantly increased the proliferation and migration of corneal epithelial cells (CECs) and promoted tear and mucin secretion in ocular surface epithelia. In addition, JBP485 accelerated corneal epithelial wound healing in vivo without inflammation and neovascularization and proved effective in treating corneal damage. The prior art data suggest that JBP485 efficiently activates the viability of CECs and can be considered as a novel treatment for various types of corneal epithelial diseases.

Epidermal growth factor (EGF) and Betacellulin (BTC) belongs to the epidermal growth factor (EGF) peptide ligand family and is characterized by a six-cysteine consensus motif that forms three intra-molecular disulfide bonds critical for binding to the ErbB receptor family. BTC binds and activates ErbB-1 and ErbB-4 homodimers and is also characterized by its unique ability to activate all possible heterodimeric ErbB-receptors. Covalently bound EGF on the surfaces actively promotes epithelial cell coverage and thus corneal epithelial regeneration and wound healing according to recent studies. Co-electrospinning of EGF and chitosan is expected to accelerate and promote wound healing.

Vascular endothelial growth factor (VEGF) plays a key role in new vessel formation, proliferation, and migration. Corneal neovascularization (CN) is a clue to various ocular diseases and can lead to corneal edema and haze with subsequent vision loss. VEGF has been found to be upregulated in such diseases, based on increased transcription of HIF-1alpha. Nowadays, it is possible to downregulate the angiogenic process using anti-VEGF agents administered by different routes. In corneal transplant procedures, it is important to consider that limbal vascularization, which can occur in the context of corneal hypoxia (in the context of infection and previous trauma) leads to an increased expression of HIF-1alpha and consequently increased VEGF release which could be antagonized by anti-VEGF agents released from the medical implant.

Recombinant human Nerve Growth Factor (rhNGF) plays a special role in corneal nerve and epithelial regeneration. Corneal nerves are key components of the physiologic system that controls ocular surface homeostasis and corneal regeneration after transplantation.

According to one embodiment, the matrix layer further comprises a surface structure supporting cell guidance of cells, such as epithelial cells, endothelial cells, limbus stem cells or keratinocytes on the outer surface of the matrix layer. To resemble the natural blueprint as far as possible and induce the desired maturation of functional tissue upon healing, topological features can be included that serve as guidance for oriented cell growth and migration of epithelial cells and keratocytes onto the corneal bio-prosthesis. This is an important feature to support long-term transparency and thus functionality after healing.

In a preferred embodiment the surface structure comprises or consists of grooves with a width of 10 to 100 μm. In combination with the chitosan as polyanion, such a structure allows the adhesion of cells and their migration along the grooves leading to a cellular anisotropy.

In a preferred embodiment, the matrix layer is produced by mold casting of a chitosan-containing solution generating a chitosan film with tailored curvature. For the use as eye-related implant, with the preferred use as cornea implant, it is necessary to provide a graft with a tailored curvature that represents the curvature of the biological target structure. In order to prepare a customized implant with a patient-tailored geometry, the mold is preferably generated by stereolithography or photolithography, whereby said mold is generated on a virtual model of the target structure, being preferably the cornea-geometry of the subject to be treated.

The use of photolithography has the advantage that the mold (and thereby also the chitosan film) can be generated with a higher resolution which is preferable for formation of surface pattern such as grooves. In case of photolithography, it is preferred to use PDMS as polymer.

**The reinforcement structure**

According to one embodiment, the reinforcement structure has a shape which is adapted to the shape of the matrix layer. The reinforcement structure is preferably completely embedded within the matrix layer so that the outer surface of the medical implant is made of chitosan as matrix material and the reinforcement structure is a purely internal structure. This provides a uniform matrix surface, which takes full advantage of the beneficial effects of the chitosan. The aforementioned adaption thus implies that the reinforcement structure is smaller in all three dimensions as compared to the matrix film layer.

In a preferred embodiment, the reinforcement structure is given in a subsection of the matrix layer and especially in those areas where a mechanical reinforcement is required, which are typically the areas for suturing the implant to the neighboring structures.

In a more preferred embodiment, the reinforcement structure has a ring structure with a central circular opening. This structure has the advantage that the central area has optimal transparency for us as a cornea graft, and the reinforcement is given for the edges of the graft, providing sufficient strength to ensure good knotability and sewability. In this context it is advantageous that the central opening of the ring structure has a diameter of between 2 and 25 mm, preferably a diameter of between 4 and 20 mm, and more preferably a diameter of between 4 and 20 mm.

According to one embodiment, the reinforcement structure the fibers of the reinforcement structure form a mechanically tailored, flexible ring consisting of interconnected loops, whereby the max. width of each loop is preferably between 100 and 1000 μm.

According to a further embodiment, the fibers of the reinforcement structure have a diameter of between 2 and 50 μm.

According to one embodiment, the fibers of the reinforcement structure comprise a polymer which is selected from the group consisting of PLLA, PLDLA, PGA, PLGA, PCL, PLLA-PCL, PVA, and combinations thereof.

In a preferred embodiment, the reinforcement structure is made from PCL. Polycaprolactone PCL is degraded by hydrolysis of its ester linkages in physiological conditions (such as in the human body) and therefore is a beneficial biomaterial for biodegradable implants. In particular it is especially interesting for the preparation of long-term implantable devices, owing to its degradation which is even slower than that of polylactide.

In a preferred embodiment, the reinforcement structure of the medical implant of the invention is a multi-layer microporous structure produced by melt-electro writing (MEW) via layer-by-layer stacking of directly-written polymer filaments.

It has been shown by the inventors that that reinforcement of the chitosan film layer and specifically for corneal applications, requires better control over the fiber diameter and geometry of the reinforcing scaffold. The inventors identified MEW as an outstanding method to produce high resolution scaffolds with complex geometry that would allow for tailoring mechanical properties of such matrix film layers.

Melt electro writing (MEW) is a high-resolution 3D printing technique that combines elements of electrohydrodynamic fiber attraction and melts extrusion. The general principle of MEW can be divided in two steps: First the molten material is extruded through a nozzle using air pressure or volumetric dispensing (e.g., piston). Secondly, a small droplet is created at the end of the nozzle. Similar to solution electro spinning, a high voltage (HV) is applied between the nozzle and the print bed. The HV exerts an electrostatic attraction on the molten material, and a so-called Taylor cone forms at the end of the nozzle. The Taylor cone is the cone-shaped deformation of a liquid surface exposed to an electric field. This deformation is the result of a balance of forces between gravity, surface tension, internal hydrostatic pressure, external gas pressure and the electric force resulting from the applied electric field. In the case of MEW, a microscale polymer filament is created at the end of the Taylor cone, which develops into a fiber and is drawn towards the collector. The applied voltage further stabilizes the polymer jet and prevents Plateau-Rayleigh instabilities, which would cause the jet splitting into droplets. The electrical field accomplishes a steady material flow and plays a major role in MEW by regulating important properties such as flight path and fiber diameter of the fiber jet. Various configurations exist were either the nozzle is charged and the print bed is grounded, or vice versa a grounded nozzle is used and a print bed is charged. Either way, a net force is established between nozzle and collector to attract the jet to the collector.

Other relevant parameters of the MEW process are the temperature of the polymer melt, the pressure or feed rate, the speed of moving elements such as the collector and/or of the print head and the working distance, defined as the distance between nozzle and collector. By adjusting these parameters, the polymer melt can steadily be extruded and directly written onto a collector. With a coordinated movement of the computer-controlled print head and also of the collector, pre-defined patterns and structures can be produced. 3D-constructs with customizable shape and microarchitecture are obtained by repetitive layer-by-layer fiber stacking. Due to the distance between nozzle and print bed, MEW is a contactless 3D printing technique, contrary to other methods such as fused deposition modelling (FDM).

According to the invention, Suture retention should be assured by applying a composite approach, which conducts MEW to generate highly precise micro-scaffolds that will be incorporated as a reinforcement structure into the chitosan matrix and allow for tailoring mechanical properties via function-by-design.

In a more preferred embodiment, the aforementioned polymers are used for MEW, whereby PCL being the most preferred polymer.

**The hydrogel layer**

In one embodiment, the medical implant comprises a further hydrogel layer on top of one side of the matrix layer. As a result, a bi-layer composite structure is obtained enabling the generation of a hierarchical structure thus mimicking the characteristics of relevant target tissues such as the cornea.

In the case of a cornea graft, the hydrogel layer can be used to mimic the Stroma's cell-laden, anisotropic 3D-struture.

In a preferred embodiment, the further hydrogel layer is situated on top of the side of the matrix layer, which represents the inner, tissue-contacting side. Thereby, the hydrogel layer can be modified to support the interaction of the medical implant with the underlying tissue, e.g., by loading with stem cells, or by addition of trophic factors.

For the special application as a cornea graft, the hydrogel layer is thus situated on the inner side of the chitosan film matrix oriented towards the corneal tissue, whereas the opposite side with the tailored curvature is facing outwards building the outermost surface of the cornea.

In a further preferred embodiment, the hydrogel layer is preferably shaped in order to fill the underlying cornea defect.

There are two preferred strategies, that can be conducted to produce the hydrogel layer as a 3D-structures: One is via mold casting and the other is 3D-printing. Notably, the stroma displays a thickness of approximately 450 to 500 $\mu$m. This thickness is relatively small for extrusion-based bio-printing, which can produce strands of >100 $\mu$m. On the other hand, printed strands can generate anisotropy, when printed in a parallel manner and gaps can be included to trigger cell migration.

In one embodiment, the hydrogel layer has a shape which is adapted to the patient-specific defect whereby it preferably is printed of struts with diameter of between 80 and 250 $\mu$m.

In a further embodiment, the hydrogel layer has a thickness of between 10 and 800 $\mu$m.

In one embodiment, the hydrogel layer is loaded with eukaryotic cells, which are preferably stroma cells.

In a further embodiment, the hydrogel layer has a transparency of at least 75% and preferably of at least 80%. The preferred use a corneal graft requires such a high transparency, which has to be given also for the hydrogel layer to ensure good eyesight.

In a preferred embodiment, the hydrogel layer is made from photo-crosslinked Gelatin methacryloyl (GelMA). This material combines an open porosity with sufficient mechanical properties. GelMA, also called photo-crosslinkable gelatin, is well suited used for biomedical applications because of its excellent biocompatibility, biodegradability, and moldability. GelMA can be used to overcome the limitations of gelatin hydrogels: 1. basic gelatin is soluble at body temperature, limiting the direct application of it as a cell carrier; 2. while the solidification of gelatin could be achieved by glutaraldehyde, it is cytotoxic, time-consuming, and highly uncontrollable. Moreover, GelMA has been constructed as various forms of scaffolds based on its characteristics: 1. 3D scaffold. 2. injectable gel. 3. bio-printed scaffold (extrusion-based and photomask-based). 4. electrospun fibrous membrane (EFM). The 3D scaffold of GelMA can be constructed precisely by photo-crosslinking, during which the photo-crosslinkable functional groups on GelMA are crosslinked with other polymers, small organic molecules, and inorganic particles to form a co-crosslinked network with reinforced mechanical properties and extra characteristics (e.g., better formability, biocompatibility) for tissue engineering.

In another preferred embodiment, the hydrogel layer is preferably generated by bioprinting. This method has the advantage that printed strands can generate anisotropy, when printed in a parallel manner and gaps can be included to trigger cell migration.

## Medical use

In a second aspect the invention provides a medical implant for use as one of the following grafts for the treatment of an ophthalmological disease:

(a) a partial cornea-replacement graft;
(b) a complete cornea-replacement graft;
(c) a conjunctiva graft;
(d) an amnion membrane-containing graft;
(e) a graft containing limbal stem cells;
(f) a graft for deep anterior lamellar keratoplasty (DALK);
(g) a graft for descemet's membrane endothelial keratoplasty (DMEK);
(h) a graft for perforating keratoplasty (PKP);
(i) a patch containing a sterile collagen membrane from bovine pericardium;
(j) a conjunctiva patch;
(k) a sclera patch.

The successful development of partial or complete cornea replacement graft represents a particular challenge: In corneal replacement, the mechanical resistance of the material plays an important role. The material must not show any lamellar splitting, both during trephination of the matrix and during suturing. When suturing the material to the recipient cornea, good suture pull-through resistance must be ensured, as well as good knotability with sufficient sinking of the knots into the tissue, and no steps must form between the matrix and the recipient bed. Furthermore, the construct must withstand the intraocular pressure (~1.3-2.8 kPa). The corneal set must also be highly transparent, at least in the central area to ensure good eyesight. Another important aspect is the natural curvature of the cornea, which is patient-specific and can easily be determined using some existing methods. This however allows for the fabrication of biomimetic grafts as Computer Aided Design (CAD) can be used to generate a virtual model of the cornea and rapid prototyping can be conducted to reproduce the patient-specific corneal geometry. To resemble the natural blueprint as far as possible and induce the desired maturation of functional tissue upon healing, topological features should be preferably included that serve as guidance for oriented cell growth and migration of epithelial cells and keratocytes onto the corneal bioprosthesis. This is an important prerequisite to support long-term transparency and thus functionality after healing. Furthermore, healing should be supported via temporarily controlled release of drugs and growth factors.
In a preferred embodiment, the medical implant of the invention can be used to treat patients with cornea diseases and limbal stem cell deficiency. Limbal stem cell deficiency (LSCD) is characterized by a loss or deficiency of the stem cells in the limbus that are vital for the repopulation of the corneal epithelium and to the barrier function of the limbus. When these stem cells are lost, the corneal epithelium is unable to repair and renew itself. This results in epithelial breakdown and persistent epithelial defects, corneal conjunctivalization and neovascularization, corneal scarring, and chronic inflammation. All of these contribute to loss of corneal clarity, potential vision loss, chronic pain and photophobia.
In a further embodiment, the medical implant of the invention can be used as an artificial wound graft for the treatment of skin disorders such as extensive wounding or trauma, burns, areas of extensive skin loss due to infection, or skin damage due to removal of skin cancer.

## Production method

In a third aspect, the invention provides a method for producing a medical implant comprising the following steps:

(a) Generating a mold, preferably by stereolithography using computer aided design to generate a virtual model of the cornea to reproduce the patient-specific cornea geometry within the mold;
(b) Optionally modifying the mold surface to generate a surface structure enabling the adhesion and migration of epithelial cells on the outer surface of the composite matrix layer, preferably consisting of grooves with a width of 10 to 100 $\mu$m;
(c) Generating a ring-shaped microporous reinforcement structure with a circular opening comprising polymer filaments, whereby said reinforcement structure is produced by melt-electro-writing;
(d) Introducing the ring-shaped reinforcement structure as generated in step (c) into the mold before casting;
(e) Casting the mold with a chitosan-containing solution to generate a fiber-reinforced film layer with a tailored curvature on the bottom side;
(f) Removing the composite structure comprising the chitosan film layer with the embedded reinforcement structure from the mold.

In a preferred embodiment, the medical implant produced by the method of the invention is a cornea graft, and more preferably a partial cornea replacement graft.
In one embodiment of the method, the reinforced chitosan film layer is overlaid, before or after step (f), on the non-curvature side with a hydrogel layer according the invention in order to generate a bilayer medical implant.

In a further embodiment, the shape of the hydrogel layer is generated in order to fill the underlying cornea defect, preferably by using the combination of 3D scan, slitlamp microscopy and photo documentation of the eye in order to capture the exact geometry of the cornea defect with subsequent 3D-printing of the hydrogel layer onto the chitosan film in order to fill the respective cornea.

DEFINITIONS

Substantially: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and/or chemical phenomena.

Approximately: The term "approximately" as used herein may be applied to modify any quantitative comparison, value, measurement, or other representation that could permissibly vary without resulting in a change in the basic function to which it is related.

A/an: As used herein the specification, "a" or "an" may mean one or more. As used herein in the claim(s), when used in conjunction with the word "comprising," the words "a" or "an" may mean one or more than one.

Or/and/or: The use of the term "or" in the claims is used to mean "and/or" unless explicitly indicated to refer to alternatives only or the alternatives are mutually exclusive, although the disclosure supports a definition that refers to only alternatives and "and/or."

The term "about" means, in general, within a standard deviation of the stated value as determined using a standard analytical technique for measuring the stated value. The terms can also be used by referring to plus or minus 5% of the stated value.

As used herein, the term "medical implant" refers to an implantable material, a prosthesis, an artificial organ, a repair device, or a patch. The site of implantation can be anywhere in or onto a body of a subject. Representative implants include both intraocular or periocular implants or patches. It includes also intraocular lenses ("LOI") and patches. Although the latter cannot be permanently implanted, during use, they are in direct contact with body tissue (e.g., the cornea) and fluids (tears). Indeed, any implant as would be apparent to one of ordinary skill in the art upon review of the present disclosure falls within the scope of the presently disclosed subject matter.

Unless otherwise stated or apparent by the particular context, the term "chitosan" as used herein includes chitosan-based moieties including chitosan itself, chitosan salts, and chitosan derivatives. Chitosan is the commonly used name for poly-[1-4]-β-D-glucosamine. It is commercially available and is chemically derived from chitin, which is a poly-[1-4]-β-N-acetyl-D-glucosamine that, in turn, is derived from the cell walls of fungi, the shells of insects and, especially, crustaceans. In the preparation of chitosan from chitin, acetyl groups are removed ("deacetylation"), and, in the chitosan used in the processes and articles disclosed herein, the degree of deacetylation is at least about 60%, and is preferably at least about 85%. As the degree of deacetylation increases, it becomes easier to dissolve chitosan in acidic medium.

Suitable chitosan-based moieties include chitosan, chitosan salts, and chitosan derivatives. Representative examples of chitosan derivatives suitable for use in the processes and articles disclosed herein include N- and O-carboxyalkyl chitosan. The number average molecular weight (Mn) in aqueous solution of the chitosan used herein is at least about 10,000.

A chitosan film may be cast from solution. If it is desired to cast a chitosan film from an aqueous solution, the chitosan is first solubilized, since chitosan is not soluble in water. Preferably, solubility is obtained by adding the chitosan to a dilute solution of a water-soluble acid. This allows the chitosan to react with the acid to form a water-soluble salt, herein referred to as a "chitosan salt" or "chitosan as the (acid anion) thereof, for example "chitosan as the acetate thereof' if acetic acid was used. Chitosan derivatives such as N- and O-carboxyalkyl chitosan that are water-soluble can be used directly in water without the addition of acid.

As used herein, the term "average molecular weight" refers to the average molecular weight of the polymer chains in a polymer composition. Average molecular weight may be calculated as either the weight average molecular weight ("Mw") or the number average molecular weight ("Mn"). Weight average molecular weight may be calculated using the equation:

$$M_w = (\Sigma_i\, N_i M_i^{\,2})\, /\, (\Sigma_i\, N_i M_i)$$

where Ni is the number of molecules having molecular weight M. Number average molecular weight may be calculated using the equation:

$$M_n = (\Sigma_i\, N_i M_i)\, /\, (\Sigma_i\, N_i).$$

The weight and number average molecular weight may be measured according to gel permeation chromatography ("GPC"), size exclusion chromatography, or other analytical methods.

As used herein, the term "reinforcement structure" refers to the filamentous structure that is incorporated into a polymeric matrix to reinforce the polymeric matrix.

In the present application, the term "polymer" refers to a macromolecular compound prepared by polymerizing monomers of the same or different types. The term "polymer" includes homopolymers, copolymers, terpolymers, interpolymers, and so forth.

As used herein, the term "filament" shall mean a material form characterized by a very high ratio of length to diameter. Filaments are produced from a variety of polymers by extruding a molten polymer material through a spinneret. During filament production, it is usually intended that filaments be substantially continuous in length, but occasionally some filaments may break, reducing their length.

The term "polymer fiber" refers to an elongated stringy material made of a natural polymer or a synthetic polymer. The polymer is referred to as "oriented" if the axis of main chains of the macromolecules are arrayed predominantly along one direction, and the axis are therefore substantially parallel to each other.

The term "biocompatible" as used herein refers to a material that, upon contact with a living element, such as a cell or tissue, causes little or no toxicity.

Analogues: As used herein the term "analogue" refers to a chemical compound that is structurally similar to another but differs slightly in composition, such as in the replacement of one atom by an atom of a different element or by a functional group.

Treatment/treating: "Treatment" or " treating" includes (1) inhibiting a disease, disorder or condition in a subject or patient experiencing or displaying the pathology or symptomatology of the disease (e.g., arresting further development of the pathology and/or symptomatology), (2) ameliorating a disease, disorder or condition in a subject or patient that is experiencing or displaying the pathology or symptomatology of the disease (e.g., reversing the pathology and/or symptomatology), and/or (3) effecting any measurable decrease in a disease, disorder or condition in a subject or patient that is experiencing or displaying the pathology or symptomatology of the disease.

Protein/polypeptide: The terms "protein," "peptide," and "polypeptide," are used interchangeably herein, and refer to a polymer of amino acid residues linked together by peptide (amide) bonds. The terms refer to a protein, peptide, or polypeptide of any size, structure, or function. Typically, a protein, peptide, or polypeptide will be at least three amino acids long. A protein, peptide, or polypeptide may refer to an individual protein or a collection of proteins. One or more of the amino acids in a protein, peptide, or polypeptide may be modified, for example, by the addition of a chemical entity such as a carbohydrate group, a hydroxyl group, a phosphate group, a farnesyl group, an isofarnesyl group, a fatty acid group, a linker for conjugation, functionalization, or other modification, etc. A protein, peptide, or polypeptide may also be a single molecule or may be a multi-molecular complex. A protein, peptide, or polypeptide may be just a fragment of a naturally occurring protein or peptide. A protein, peptide, or polypeptide may be naturally occurring, recombinant, or synthetic, or any combination thereof.

BRIEF DESCRIPTION OF THE FIGURES

To illustrate the technical features of embodiments of the present invention more clearly, the accompanying Figures provided for describing the embodiments are introduced briefly in the following. The accompanying figures in the following description are merely some embodiments of the present invention, modifications on these embodiments are possible without departing from the scope of the present invention as defined in the claims.

FIG. 1 shows the procedure of an anterior lamellar keratoplasty (DALK): Dissection of the diseased anterior stromal tissue, which is replaced by a lamellar graft and fixed with sutures (1-8). Lamella shall be clear, fit well into the receiver transplant bed without steps can be sutured well circularly, do not show cracking without subsequent material defect, withstand tensile stress and have inherent elasticity.

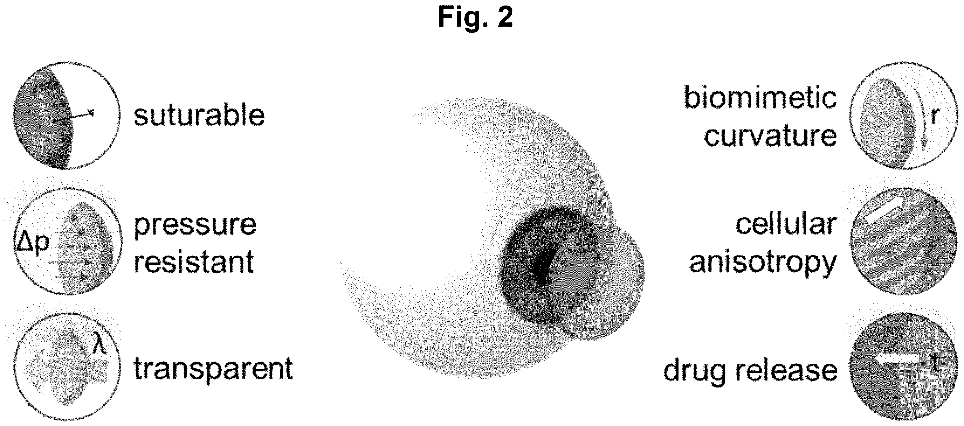

FIG. 2 shows the design criteria that are pre-defined for tissue engineering of stromal cornea replacement membranes and are fulfilled by the cornea graft of the invention.

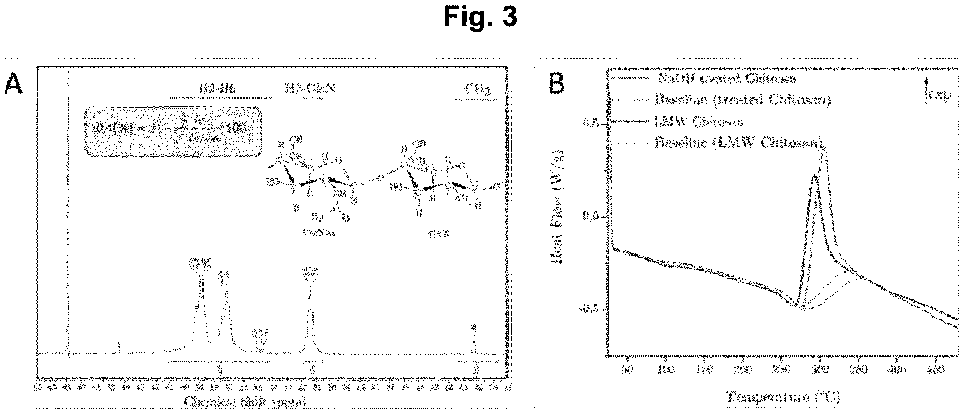

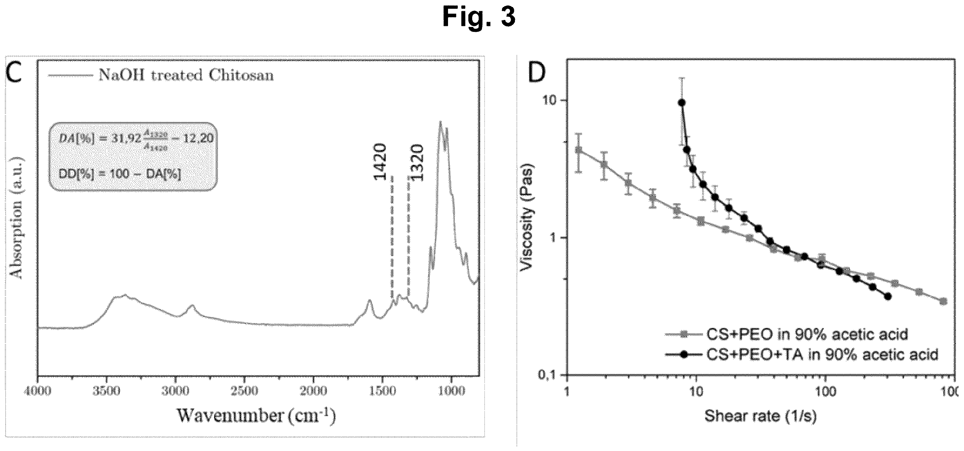

FIG. 3 shows exemplary measurements that were conducted to characterize commercially available chitosan and determine the impact of further deacetylation steps. The methods applied are $^1$HNMR (A), DSC (B), FT-IR (C). Shear rheology (C) was used to determine the impact of additives to solution properties such as viscosity, which is relevant for processing parameterization.

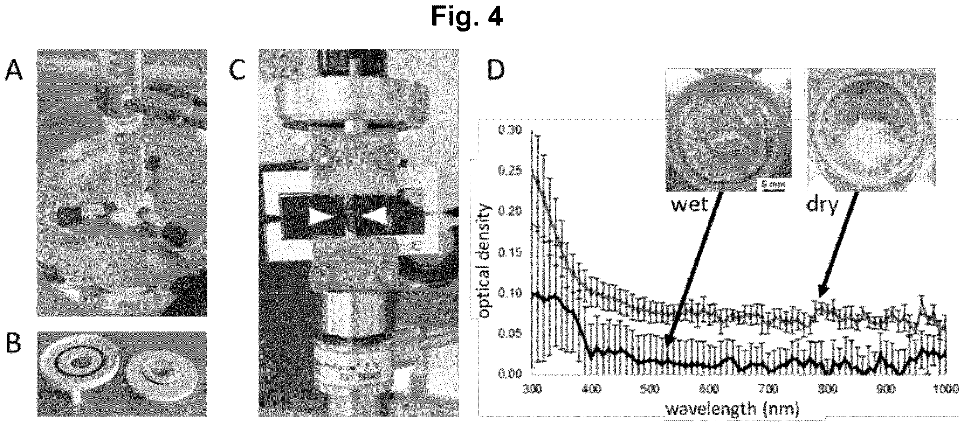

FIG. 4 shows burst pressure tests with chitosan films were performed using a water column with a height of 100 cm thus corresponding to five times the intraocular pressure (75 mmHg) (A). Therefore, samples were clamped in a custom-built 3D-printed confinement (B). Tensile testing was performed using a Bose Electroforce test setup (C). Optical density of dry and wet (PBS) chitosan films was measured photometrically and revealed good transparency in the visible wavelength range (D).

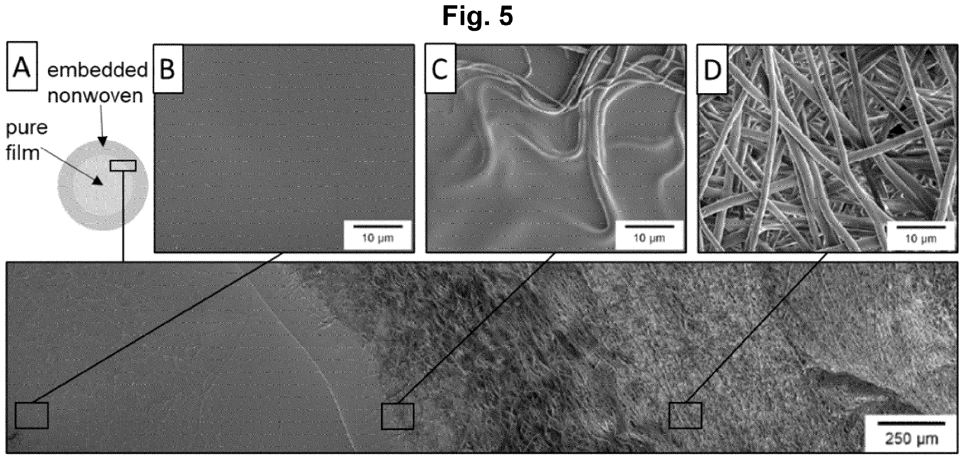

Fig. 5 shows a schematic structure and SEM-image of a fiber-reinforced chitosan membrane (A), whereby the reinforcement structure is an electrospun nonwoven embedded as a lateral ring structure within the chitosan film. In the middle part of the membrane where no fibers are present, the chitosan film displays a smooth surface (B), whereas in the transition zone from plain to reinforced membrane, embedded fibers are visible (B) and on the outer part, fibers protrude the membrane (C).

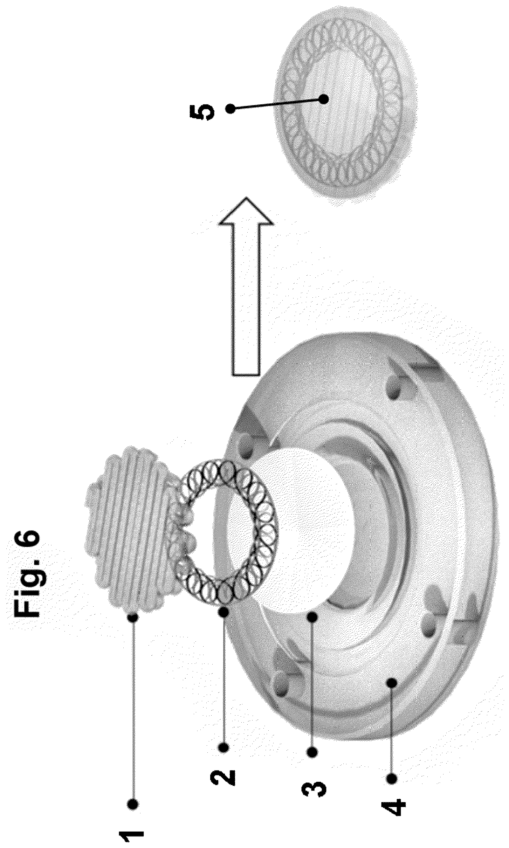

Fig. 6.    Shows a schematic composition for a cornea replacement graft representing one embodiment of the invention. MEW is applied to produce a PCL-scaffold as mechanical support for a chitosan film, that are cast into SLA-printed curved molds with microgrooves. Cell-laden bio-inks can subsequently be 3D-printed into the mold to generate a cell-containing hydrogel layer on top of the fiber-reinforced chitosan film.

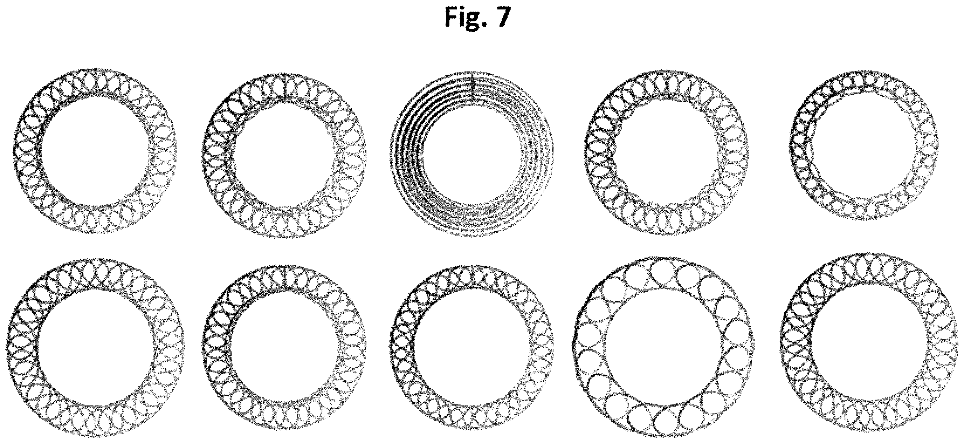

Fig. 7.    Shows several designs for the reinforcement structure of the present invention as generated by MEW.

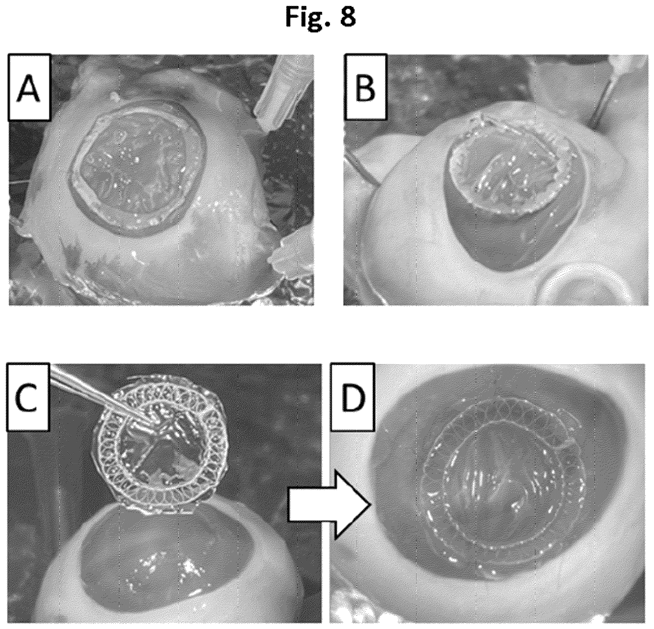

Fig. 8    shows suture tests on pig eyes applying chitosan films that were reinforced with an electrospun nonwoven (A), a 3D-printed scaffold (B) or a melt-electrowritten microscaffold (C, D), respectively.

LIST OF REFERENCE SIGNS

1 Cell-loaded hydrogel layer
2 Reinforcement structure generated by MEW
3 Chitosan film matrix
4 Mold
5 Medical implant as fiber-reinforced film with cell-loaded hydrogel layer

EXAMPLES

The following examples are for illustrative purpose only and are not to be construed as limiting this invention in any manner.

## 1. Preparation of a corneal graft according to the invention

### 1.1 Preparation of a chitosan film layer by mold casting

Chitosan films were cast into square (17.5x17.5 mm) wells with a glass bottom. For the low molecular weight chitosan (Merck, Darmstadt, Germany), a 1% (w/v) solution in 50 mM acetic acid was used, whilst for the high molecular weight (Merck, Darmstadt, Germany) a 0.5% (w/v) was used, in both cases 2 ml were cast. The films were then left to dry overnight in the fume hood. The dried film was subjected to the following film post treatment: The dried samples were treated with 1 ml 100 mM NaOH for 30 min, washed once with PBS and thrice with $H_2O$. The films were then removed from the wells and left to dry or used further without drying.

### 1.2 Preparation of a reinforcement structure by MEW

The reinforcement structure was produced by melt electro writing using an in-house custom built melt electro writing device, essentially representing the device as described in Reizabal et al. (Additive Manufacturing 2023, 17, 103604) and polycaprolactone as polymer with the following parameters:

| | |
|---|---|
| Temperature of the PCL melt: | 95°C |
| Needle: | 22G |
| Pressure: | 1 bar |
| Printing distance: | 2.4 mm |
| Voltage: | 2.5 kV |

Suitable designs for the reinforcement structure are shown in Figure 7.

### 1.3 Introduction of the reinforcement structure into the freshly casted chitosan film

First the PCL scaffolds were placed into the wells and covered with 500 μl of the $H_2O$ solution and removed immediately thereafter to hydrate the scaffold and remove microbubbles. Thereafter 2 ml of the chitosan solution was applied, and the scaffolds pressed to the bottom. The samples were then degassed in a desiccator under vacuum. The floating scaffolds were pushed to the bottom of the well and the samples left to dry under the fume hood. Here maybe also film post treatment: The dried samples were treated with 1 ml 100 mM NaOH for 30 min, washed once with PBS and thrice with $H_2O$. The films were then removed from the wells and left to dry or used further without drying.

### 1.4 Deposition of a cell-loaded hydrogel layer on top of the chitosan film

The porcine GelMA (synthesized as described by Loessner et al. (Nature Protocols 2016; 11, 727-746) using ~300 g Bloom, Type A, Merck KGaA, Darmstadt, Germany) solution was made by dissolving 15 % (w/v) GelMA in PBS at 40-50 °C. Once the GelMA had been dissolved, 0.1 % (w/v) of the photoinitiator Lithium phenyl (2,4,6-trimethylbenzoyl) phosphinate (LAP) were added.
For the patterning, digital light processing (DLP) printed constructs were made with 100 or 50 μm grooves and an eye curved shape. The shapes were printed with the print layers parallel to the grooves for the best resolution. Complementary press pieces to the curvature were also printed so that the film can be put onto these and pressed into the GelMA, resulting in the right curvature.

The grooved casting chamber was coated with a plant oil-based mold removal spray. The GelMA was heated to 40 °C and 60 µl were applied into the casting chamber, trying to distribute it as evenly as possible along the entire grooved structure. Immediately after the GelMA application, film was pressed onto the GelMA layer, the press removed and the casting chamber illuminated with 365 nm UV light for 2 min, the cast was then gently removed using tweezers.

## 2. Characterisation of chitosan and implant generated therewith

### 2.1 Analysis of the chitosan or blend thereof as starting material

The chitosan is analysed for the degree of deacetylation (DD) by using 1 H NMR. A typical 1 H NMR spectrum of chitosan is shown in Figure 3A, and obtained assignments and chemical shifts of the 1H NMR signals are given as follows according to Figure 3A: The signal at 2.0-2.1 ppm corresponds to the three protons of N-acetyl glucosamine (GlcNAc) residues, and it possess the highest resolution and, so, is typically used as reference for quantitative determinations.

The H2 proton of glucosamine (GlcN) units presents a characteristic peak at 3.1-3.2 ppm with high resolution, while the nonanomeric protons (H1) resonate around 4.6-4.8 ppm with low resolution and overlapped with the signal of water. The rest of the signals (H3, 4, 5, 6) show a broad envelope of peaks between 3.5 and 4 ppm. Based on the peaks described above, the DD can be calculated according the following formula:

$$DD = \left[ 1 - \frac{1/3 \times I_{\text{N-COCH}_3}}{1/6 \times I_{\text{(H2-H6)}}} \right] \times 100$$

Here $I_{\text{N-COCH3}}$ is the integral of the signal corresponding to H7 methyl protons of GlcNAc units and $I_{\text{(H2-H6)}}$ is the sum of the integrals of H2, H3, H4, H5, and H6.

The chitosan film as generated in Example 1 above is tested for optical properties as shown in **Figure 4D** to correlate transparency with film thickness, topological features, and degree of deacetylation.

The viscosity of the chitosan or chitosan blend is determined as follows: Rheological tests are conducted using a shear rheometer (e.g. Discovery HR-2 rheometer (TA Instruments) with a 25 mm plate-plate geometry, maintaining a 200 µm gap. The temperature is controlled at 25°C via a Peltier element in the lower plate, and a solvent trap is used to prevent evaporation. Samples are preconditioned for 60 seconds at a shear rate of 10 s$^{-1}$ to eliminate loading-induced tensions and ensure uniform temperature. Amplitude sweeps determine the linear viscoelastic (LVE) limit according to ASTM D7175 and DIN 51810-2, measuring storage and loss moduli at 10 rad/s across 0.01% to 200% deformations. Frequency sweeps are performed at 0.1% deformation over a frequency range of 0.1 to 100 rad/s to measure storage modulus, loss modulus, and complex viscosity. Viscosity is measured using flow ramps, increasing the shear rate logarithmically from 0.1 s$^{-1}$ to 100 s$^{-1}$. Each test is repeated at least three times, and average values with standard deviations are calculated. Test tests allow for predictions of the flow behaviour upon fabrication of chitosan films.

### 2.2 Analysis of the chitosan film layer prepared according Example 1

The pressure resistance of the casted chitosan films was determined as follows: Casted chitosan films with different thickness (10-80 µm) were generated and subjected to a surface pressure of approximately five times the intraocular pressure (10 kPa). Therefore, the samples were placed in a water column of 1 m height and long-term pressure-resistance was examined over a period of 7 days. We could show, that besides a few of the thin samples, all membranes could withstand the pressure.

The tensile of the casted chitosan films was determined as follows: To evaluate the mechanical properties of the materials, they underwent tensile testing on the ElectroForce 3220 (Bose Corp./TA Instruments) instrument. The 2.49 N (5 lbf) measuring cell was used. For preparation, the samples were cut into strips approximately 2 mm wide. These sections were placed on rectangular paper frames with a gap of 12 mm and secured with superglue. The frames were then clamped in the apparatus, and the sides were cut. The sample chamber was brought to a humidity of ≥ 97%. After an incubation time of 2:30 min, the tensile measurement was started. The WinTest software was set to a maximum displacement of 12 mm and a speed of 0.1 mm/s.

Tensile testing resulted in a tensile strength and extensibility of 14 kPa and 2 % in the dry and 1.6 kPa and 45% in the wet state. Strikingly, material failure was always accompanied with crack propagation from the sample's edges, which explains the superior material properties in the water column test, where no free edges were available for crack formation. Consequently, it was not surprising, that sewability-test displayed very weak performance irrespective of the film thickness as the thread would tear out easily.

### 2.3 Analysis of the chitosan based membranes for their surgical use as cornea grafts

Promising chitosan-based membranes as cornea grafts will be analysed by extended analytical tools to demonstrate the practical applicability of such membranes for clinical use. To evaluate their surgical applicability in the cornea, the membranes will be prepared using a trephine analogous to human corneal trephination and fixed with 10-0 nylon sutures to/in the cornea of enucleated porcine eyes and tested and analysed for the following criteria.

- Trepanning ability: Evaluation criteria: straight cut edges, no pressure-induced splitting of the matrix into individual fibers (optical testing with the help of a specially created evaluation scale, reference for this human corneas).

- Sewability: Evaluation criteria: Tensile strength, handling of the matrix during suturing (documentation with the aid of a specially developed evaluation scale, reference for this is human corneas).

- Knotability: Evaluation criteria: Tensile strength, sinking of the knot into the tissue, adaptation of matrix and recipient tissue, step formation (evaluation with the help of an evaluation scale, reference for this are human corneas).

Furthermore, advanced transparency measurements should be performed in accordance to clinically relevant quality criteria including total transmission, haze, and clarity. Therefore, illumination method with writing sharpness measurement, Densitometry measurement (Oculus Pentacam) and Shape stability and refraction/Topography measurement (High resolution VAA SL-OCT, Casia2/Anterion) will be applied.

Ex vivo experiments showed that micro-scaffolds produced via MEW display significantly better properties when sutured onto pig eyes (Figure 8 C, D) as compared to the chitosan grafts reinforced with a non-woven ring structure made from electrospun polymer fibers (Figure 8 A) or a 3D-printed scaffold produced via fused deposition modelling (FDM) (Figure 8 B).

## 3. Preparation of alternative reinforcement structures

The chitosan film layer was reinforced by two alternative reinforcement structures:

a) Non-woven ring structure made from electrospun polymer fibers
b) 3D-printed scaffold produced via fused deposition modelling (FDM)

Chitosan film-based cornea grafts were reinforced by the above two structures and analysed with regard to their surgical use.

## 3.1 Non-woven ring structure made from electrospun polymer fibers

Round shaped non-woven mats were produced with a diameter of 1 cm and a 0.8 cm hole in the middle.

The samples were produced via electrospinning of polycaprolactone (PCL) and embedded into chitosan films resulting in fiber reinforcement at the edges of the membranes and transparency at the middle part where not fibers were present. Scanning electron microscopy showed the successful combination of PCL-fibers and chitosan films and initial suture test confirmed improved tear-out resistance. To further analyse their suitability to be attached to biological tissue, samples were sutured onto pig eyes. These experiments demonstrated that membranes reinforced with electrospun fibers are difficult to handle due to their soft and flexible properties (Figure 8, A) and thus are not optimally suited for a use a cornea graft. However, this type of reinforcement-structure might be suitable for other applications (e.g., skin grafts or patches).

## 3.2 3D-printed scaffold produced via fused deposition modelling (FDM)

To improve shape fidelity, we also tested reinforcement with different 3D-printed scaffolds produced via fused deposition modelling (FDM). The FDM scaffolds were produced as follows: A PCL filament (Facilan™ PCL 100, 3D4makers, Haarlem, the Netherlands) was used for 3D printing at 130 °C nozzle temperature and 60 °C print bed temperature using an Ultimaker S5 (Ultimaker, Zaltbommel, the Netherlands). When testing the resulting reinforced chitosan membranes, it turned out that the membranes were too stiff (Figure 8, B) for this particular use as cornea graft. However, this type of reinforcement-structure might be suitable for other applications (e.g., skin grafts or patches).

## Claims

1. A medical implant, comprising:

    (a) a matrix layer comprising chitosan; and
    (b) a reinforcement structure comprising polymer filaments or polymer fibers,

    whereby the reinforcement structure is incorporated into the matrix layer.

2. The medical implant according to claim 1, **characterized in that** the chitosan is a low molecular weight (LMW) chitosan with a Mw of less than 150 KDa, having preferably a degree of deacetylation of at least 95%, more preferably of at least 97%, and even more preferably of at least 99%, as determined by $^1$H-NMR analysis.

3. The medical implant according to claim 1 or 2, **characterized in that** the matrix layer comprises:

    (a) a blend of chitosan and collagen which is crosslinked, preferably by use of glutaraldehyde, NaOH, or carbodiimides such as 1-Ethyl-3-(3-dimethylaminopropyl) carbodiimide (EDC);
(b) a crosslinked chitosan, preferably crosslinked by use of glutaraldehyde;

whereby the surface of the matrix layer is preferably coated with fibrinogen or an RGD-containing peptide, more preferably with the peptide RGDS via carbodiimide reaction.

4. The medical implant according to claims 1 to 3, **characterized in that** the matrix layer has one or more of the following characteristics:

(a) a chitosan concentration of between 50 and 99 wt.%;
(b) a thickness of between 10 and 800 μm;
(c) a transparency of at least 75% and preferably of at least 80%;
(d) a burst pressure of at least 5000 Pa and preferably of at least 10000 Pa;
(e) further comprises an additive such as polyethylene oxide, silk proteins, cellulose nanofibrils, or tannic acid;
(f) further comprises a drug or a bioactive molecule, preferably selected from the group consisting of curcumin, collagen I, EGF, betacellulin, anti-VEGF, rho-associated coiled-coil-containing protein kinase (ROCK) inhibitor, cyclo-trans-4-L-hydroxypropyl-L-serine (JPB485), human recombinant nerve growth factor (NGF), Insulin growth factors, Bosentan, Sparsentan, 4-Hydroxytamoxifen, Tamoxifen and estrogen receptor β (ER-β) agonist LY500307, and combinations thereof;
(g) further comprises a surface structure supporting cell guidance of epithelial cells, endothelial cells, limbus stem cells or keratinocytes on the outer surface of the matrix layer, preferably consisting of grooves with a width of 10 to 100 μm.

5. The medical implant according to any of claims 1 to 4, **characterized in that** the matrix layer is produced by mold casting of a chitosan-containing solution generating a chitosan film with tailored curvature, whereby the mold is preferably generated by stereolithography or photolithography.

6. The medical implant according to any of the above claims, **characterized in that** the reinforcement structure has one or more of the following characteristics:

(a) a shape which is adapted to the shape of the matrix layer and preferably has a ring structure with a central circular opening, whereby the opening has a diameter of between 2 and 25 mm;
(b) a diameter of between 3 and 30 mm;
(c) a thickness of between 10 and 800 μm;
(d) the fibers of the reinforcement structure form a mechanically tailored, flexible ring consisting of interconnected loops, whereby the max. width of each loop is preferably between 100 and 1000 μm;
(e) the fibers of the reinforcement structure have a diameter of between 2 and 50 μm.

7. The medical implant according to any of the above claims, **characterized in that** the fibers of the reinforcement structure comprise a polymer which is selected from the group consisting of PLLA, PLDLA, PGA, PLGA, PCL, PLLA-PCL, PVA, and a combination thereof, whereby the polymer preferably is PCL.

8. The medical implant according to any of the above claims, **characterized in that** the reinforcement structure is a multi-layer microporous structure produced by melt-electro writing (MEW) via layer-by-layer stacking of directly-written polymer filaments using preferably a polymer according to claim 7.

9. The medical implant according to any of the above claims, **characterized in that** the medical implant comprises a further hydrogel layer on top of the matrix layer, on the side that represents the inner side whereby the hydrogel layer is preferably shaped in order to fill the underlying cornea defect.

10. The medical implant according to claim 9, **characterized in that** the hydrogel layer has one or more of the following characteristics:

(a) a shape which is adapted to the patient-specific defect, whereby the hydrogel layer is preferably printed of struts with diameter of between 80 and 250 μm;
(b) a thickness of between 10 and 800 μm;
(c) is loaded with eukaryotic cells, which are preferably stroma cells;
(d) a transparency of at least 75% and preferably of at least 80%;
(e) is made from photo-crosslinked Gelatin methacryloyl (GelMA);

whereby the hydrogel layer is preferably generated by bioprinting.

11. The medical implant according to any of the above claims for use as one of the following grafts for the treatment of an ophthalmological disease:

(a) a partial cornea-replacement graft;

(b) a complete cornea-replacement graft;

(c) a conjunctiva graft;

(d) an amnion membrane-containing graft;

(e) a graft containing limbal stem cells;

(f) a graft for deep anterior lamellar keratoplasty (DALK);

(g) a graft for descemet's membrane endothelial keratoplasty (DMEK);

(h) a graft for perforating keratoplasty;

(i) a patch containing a sterile collagen membrane from bovine pericardium;

(j) a conjunctiva patch;

(k) a sclera patch.

12. The medical implant according to any of the claims 1 to 10 for use as an artificial wound graft for the treatment of skin disorders such as extensive wounding or trauma, burns, areas of extensive skin loss due to infection, or skin damage due to removal of skin cancer.

13. A method for producing a medical implant according to any of claims 1 to 8, comprising the following steps:

(a) Generating a mold, preferably by stereolithography using computer aided design to generate a virtual model of the cornea to reproduce the patient-specific cornea geometry within the mold;

(b) Optionally modifying the mold surface to generate a surface structure enabling the adhesion and migration of epithelial cells on the outer surface of the matrix layer, preferably consisting of grooves with a width of 10 to 100 $\mu$m;

(c) Generating a ring-shaped microporous reinforcement structure with a circular opening comprising polymer filaments or polymer fibers, whereby said reinforcement structure is produced by melt-electro-writing;

(d) Casting the mold with a chitosan-containing solution to generate a film layer with a tailored curvature on the bottom side;

(e) Introducing the ring-shaped reinforcement structure as generated in step (c) into the casted film layer of step (d) before said film layer has been solidified;

(f) Removing the composite structure comprising the chitosan film layer with the embedded reinforcement structure from the mold.

14. The method according to claim 13, whereby, before or after step (f), the reinforced chitosan film layer is overlaid on the non-curvature side with a hydrogel layer according to claims 9 or 10 in order to generate a bilayer medical implant.

15. The method according to claim 14, whereby the shape of the hydrogel layer is generated in order to fill the underlying cornea defect, preferably by using the combination of 3D scan, slitlamp microscopy and photo documentation of the eye in order to capture the exact geometry of the cornea defect with subsequent 3D printing of the hydrogel layer onto the chitosan film in order to fill the respective cornea.

## Fig. 1

a = stroma replacement *** = stroma replacement

**Fig. 2**

suturable

pressure resistant $\Delta p$

transparent $\lambda$

biomimetic curvature $r$

cellular anisotropy

drug release $t$

# Fig. 3

EP 4 670 748 A1

# Fig. 3

**Fig. 4**

Fig. 5

EP 4 670 748 A1

Fig. 6

Fig. 7

# Fig. 8

Europäisches
Patentamt

European
Patent Office

Office européen
des brevets

## EUROPEAN SEARCH REPORT

**Application Number**

EP 24 18 4995

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/154356 A1 (COSTELLA LAUREN ANNE [US] ET AL) 27 May 2021 (2021-05-27)<br>* abstract *<br>* paragraph [0003] *<br>* paragraph [0006] *<br>* paragraph [0008] *<br>* paragraph [0018] *<br>* paragraph [0021] *<br>* paragraph [0024] *<br>* paragraph [0033] *<br>----- | 1-15 | INV.<br>A61L27/48<br>A61L27/54 |
| | | | **TECHNICAL FIELDS SEARCHED (IPC)**<br><br>A61L |

The present search report has been drawn up for all claims

| Place of search | Date of completion of the search | Examiner |
|---|---|---|
| The Hague | 7 January 2025 | Fort, Marianne |

EPO FORM 1503 03.82 (P04C01)

## ANNEX TO THE EUROPEAN SEARCH REPORT
## ON EUROPEAN PATENT APPLICATION NO.

EP 24 18 4995

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2025

| Patent document cited in search report | Publication date | Patent family member(s) | Publication date |
|---|---|---|---|
| US 2021154356 A1 | 27-05-2021 | CN 112118874 A | 22-12-2020 |
| | | EP 3773766 A1 | 17-02-2021 |
| | | US 2021154356 A1 | 27-05-2021 |
| | | WO 2019199630 A1 | 17-10-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **REIZABAL et al.** *Additive Manufacturing*, 2023, vol. 17, 103604

- **LOESSNER et al.** *Nature Protocols*, 2016, vol. 11, 727-746